# FASCICULE DE BREVET EUROPEEN

(11) **EP 0 792 179 B1**
(45) Date de publication et mention de la délivrance du brevet: **26.05.1999**
(21) Numéro de dépôt: 95940323.9
(22) Date de dépôt: 16.11.1995
(51) Int. Cl.: A61N 1/30

(54) **DISPOSITIF D'ADMINISTRATION TRANSDERMIQUE DE MEDICAMENTS POUR LE TRAITEMENT DE L'IMPUISSANCE ERECTILE MASCULINE**
VORRICHTUNG ZUR TRANSDERMALEN VERABREICHUNG VON MEDIKAMENTEN ZUR BEHANDLUNG DER MÄNNLICHEN SEXUELLEN IMPOTENZ
DEVICE FOR THE TRANSDERMAL ADMINISTRATION OF MEDICAMENTS TO TREAT THE MALE ERECTILE IMPOTENCE

(30) Priorité: 16.11.1994 FR 9413716
(43) Date de publication de la demande: 03.09.1997
(73) Titulaire: LABORATOIRES D'HYGIENE ET DE DIETETIQUE L.H.D. Société Anonyme dite:, 75008 Paris (FR)
(72) Inventeur: MILLOT, Philippe, F-21000 Dijon (FR); LAMOISE, Michel, F-21110 Bessey-les-Citeaux (FR)
(74) Mandataire: Colas, Jean-Pierre
(86) Numéro de dépôt international: FR9501510
(87) Numéro de publication internationale: WO9614897

(56) Documents cités:
- WO-A-86/07269
- WO-A-94/15527
- US-A- 4 279 256
- US-A- 4 585 005

## Description

La présente invention est relative à un dispositif d'administration transdermique d'un médicament pour le traitement de l'impuissance érectile masculine comme décrit dans le préambule de la revendication 1. Le document WO-A-94/15527 compose le préambule de la revendication 1.

L'impuissance organique masculine, c'est-à-dire l'anérection, est le plus souvent d'origine iatrogéne, hormonale, vasculaire ou psychologique. Jusqu'à présent, on a tenté de traiter cette affection, dans certains cas, par injection de papavérine dans les corps caverneux. Cette technique présente divers inconvénients L'administration de papavérine par piqûre est, d'une part, douloureuse et d'autre part dangereuse. En effet, elle peut provoquer à terme des lésions irréversibles telles que l'apparition de plaques ou nodules fibreux dans les corps caverneux. Elle provoque aussi parfois des érections exagérément prolongées qu'il faut traiter par d'autres injections pour les réduire. On a aussi proposé de traiter l'anérection par des moyens purement mécaniques, constitués par un dispositif pneumatique à vide que l'on installe autour du pénis pour en provoquer l'érection. Un anneau bloque ensuite toute fuite de sang hors des corps caverneux.

Il est clair que ces procédés de traitement connus ne sont pas satisfaisants du fait de leur caractère traumatisant et/ou dangereux et de la lourdeur de leur mise en oeuvre.

La présente invention a donc pour but de réaliser un dispositif de traitement de l'impuissance érectile masculine qui ne présente aucun de ces inconvénients et qui assure donc un traitement indolore, facile et sans danger de cette affection.

On atteint ces buts de l'invention, ainsi que d'autres qui apparaîtront à la lecture de la description qui va suivre avec un dispositif d'administration transdermique d'un médicament pour le traitement de l'impuissance érectile masculine comme décrit dans la revendication 1.

Grâce à l'expansibilité du bracelet, on évite que le gonflement progressif des corps caverneux pendant le traitement ne provoque un étranglement du pénis par le bracelet et on assure un contact ferme et continu du réservoir sur le pénis, nécessaire à l'administration transdermique du médicament.

Le dispositif comprend en outre des moyens, également supportés par le bracelet, pour assurer une assistance par électrophorèse ou ionophorèse du passage du médicament à travers la peau du patient. Il est alors possible de commander et de contrôler le flux de médicament passant à travers cette peau. Cette commande et ce contrôle peuvent alors être affinés, suivant l'invention, à l'aide d'un capteur fixé sur le bracelet et sensible à l'expansion de celui-ci pour fournir un signal représentatif de cette expansion aux moyens d'assistance, de manière à assurer une commande de ceux-ci en fonction de l'expansion du bracelet et donc du pénis, sous l'effet du gonflement des corps caverneux.

On comprend que, grâce à ce capteur il est possible d'arrêter pratiquement l'administration du médicament dès qu'une érection suffisante est obtenue, ce qui évite toute sur-administration dangereuse. Il est possible aussi de commander l'administration du médicament suivant un programme temporel prédéterminé, comme cela est classique dans l'administration transdermique de médicament assistée par électrophorèse ou ionophorèse.

Suivant un mode de réalisation particulier de l'invention, le capteur est constitué par une jauge de contraintes fixée sur le bracelet de manière à être sensible à l'expansion de celui-ci.

D'autres caractéristiques et avantages du dispositif suivant la présente invention apparaîtront à la lecture de la description qui va suivre et à l'examen du dessin annexé dans lequel :
- la figure 1 est une vue éclatée d'un premier mode de réalisation du dispositif suivant l'invention et la figure 2 représente schématiquement ce mode de réalisation après l'assemblage de ses composants,
- la figure 3 est une vue éclatée d'un deuxième mode de réalisation du dispositif suivant l'invention et la figure 4 représente schématiquement ce mode de réalisation après l'assemblage de ses composants,
- les figures 5 et 6 sont des vues schématiques faisant apparaître des électrodes des dispositifs des figures 2 et 4 respectivement, développées dans un plan, et
- les figures 7 et 8 sont des diagrammes fonctionnels de deux modes de réalisation de moyens utilisés pour délivrer un courant électrique d'assistance électrophorétique ou ionophorétique d'administration du médicament, utilisables dans les dispositifs des figures 2 et 4.

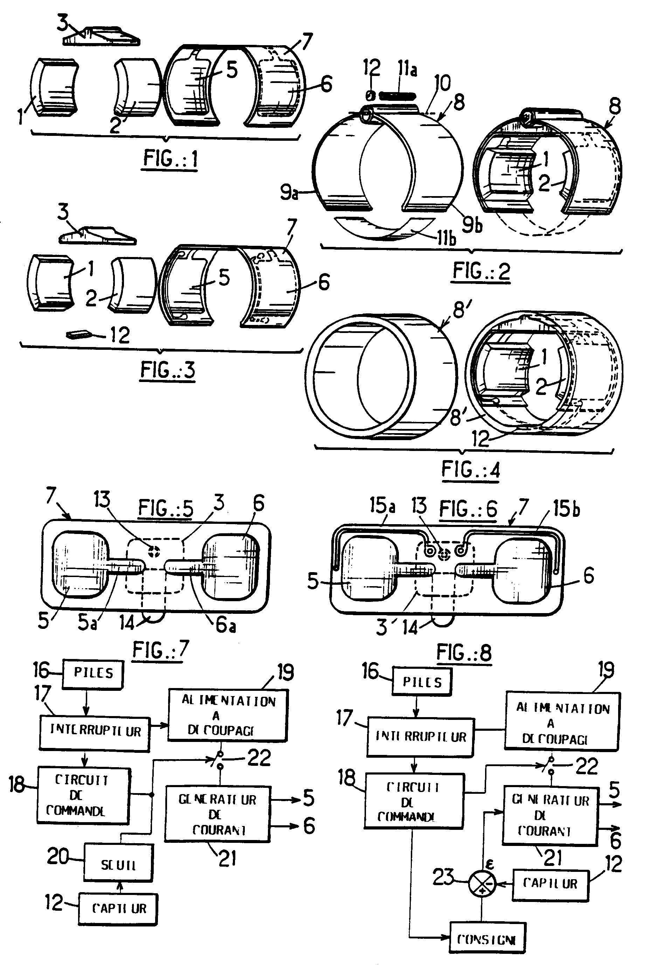

On se réfère à la figure 1 du dessin annexé où l'on a représenté schématiquement, en vue éclatée, un premier mode de réalisation du dispositif suivant l'invention. Celui-ci comprend essentiellement, à titre d'exemple, au moins un réservoir 1 d'un médicament dont le principe actif est une molécule propre à lutter contre l'impuissance érectile de l'homme. Parmi les molécules ou classes de molécules utilisables dans cette application, on peut citer à titre d'exemple, la moxisylite et ses sels, l'agoniste du polypeptide intestinal vasoactif, la papavérine seule ou associée à la phentolamine, l'agoniste de la prostaglandine E1, la prostaglandine E1, l'agoniste de l'adrénorécepteur Béta 2, l'antagoniste de la hydroxytryptamine 2, l'antagoniste de l'adrénorécepteur Alpha 2, l'agoniste de la dopa d2, l'inhibiteur de la capture de l'hydroxytryptamine, la phénoxybenzamine, l'acétylcholine, la sydnonimine et dérivés, la testostérone, les vasodilatateurs périphériques, le minoxidil et ses dérivés, l'alprostadil et des récepteurs histaminiques H2 et H3 agoniste comme l'impromidine.

Le réservoir 1 peut être constitué classiquement par une couche d'un hydrogel hydraté avec une solution d'un principe actif constitué par l'une des molécules précitées, par exemple, juste avant la mise en place du dispositif. Le réservoir 1 pourrait aussi être constitué d'un produit spongieux ou non tissé, imprégné de ladite solution. Avantageusement, le dispositif peut comprendre un deuxième réservoir 2 constitué comme le premier. Les deux réservoirs sont conformés et dimensionnés pour s'appliquer sur la peau du pénis, lors d'un traitement. Le dispositif comprend encore un module électronique 3, intégrant une source d'énergie électrique constituée d'une ou plusieurs piles et des moyens alimentés par cette source pour établir et commander un courant passant d'un réservoir à un autre, sous la peau du pénis, pour assister ionophorétiquement le passage sous cette peau d'une forme ionisée de la molécule du principe actif. En choisissant le sens du courant, l'administration du principe actif s'effectue sélectivement à partir de l'un ou l'autre des deux réservoirs.

Bien entendu, le dispositif pourrait comprendre un seul réservoir, le courant passant alors sous la peau du patient entre ce réservoir et une électrode adjacente "de retour" plaquée contre la peau du patient.

Pour mettre les réservoirs 1, 2 du dispositif de la figure 1 en contact électrique avec la source du courant d'assistance, on plaque les réservoirs contre deux électrodes 5, 6 respectivement qui prennent par exemple la forme de couches conductrices déposées sur un film souple 7 par tout moyen connu. Le film 7, sur lequel on rapporte et on fixe en repérage le module 3 et les réservoirs 1 et 2, est lui-même fixé, suivant la présente invention, dans un bracelet 8, contre la face interne de ce bracelet, suivant l'assemblage final représenté à la figure 2. Il apparaît que le bracelet 8 est constitué de deux parties 9a, 9b rigides ou semi-rigides, en forme de coquilles cylindriques articulées l'une sur l'autre autour d'un axe 10. Les deux coquilles sont chargées l'une vers l'autre soit par un ressort hélicoïdal 11a convenablement disposé sur l'axe 10, soit par une bandelette élastique 11b reliant les extrémités des coquilles 9a, 9b qui ne sont pas articulées l'une sur l'autre.

Bien entendu, si on renonce à toute assistance ionophorétique, le dispositif suivant l'invention peut se réduire au bracelet 8 et à un ou plusieurs réservoirs tels que 1 et 2.

On comprend qu'en écartant manuellement les deux coquilles l'une de l'autre, il est possible de passer le dispositif sur le pénis, jusqu'à sa racine. Dans cette position, après relâchement des coquilles, les moyens élastiques utilisés (11a ou 11b), plaquent le ou les réservoirs 1 et 2 contre la peau du pénis, avec une charge élastique légère mais suffisante pour assurer le maintien du dispositif dans cette position sans étranglement du pénis, conformément à l'un des objectifs essentiels de la présente invention.

Il suffit ensuite d'activer le module électronique 3 en agissant sur un interrupteur (non représenté) pour qu'un courant électrique passe entre les électrodes 5 et 6, courant dont le module commande l'intensité pour assurer classiquement l'administration du médicament chargé dans un des réservoirs.

Suivant l'invention, le dispositif comprend alors un capteur 12, monté par exemple au niveau de l'articulation des deux coquilles 9a, 9b pour être sensible à l'écartement relatif de ces coquilles. Comme celui-ci est fonction du gonflement des corps caverneux du pénis, on dispose alors d'une mesure de ce gonflement, mesure qui est prise en compte par le module électronique pour régler ou arrêter le flux de médicament à travers la peau, comme on le verra plus loin en liaison avec les figures 7 et 8.

Le capteur utilisé à cet effet peut être constitué par une jauge extensométrique, par exemple la jauge type 02 UW commercialisée par la société des Etats-Unis d'Amérique VISHAY, qui présente un très faible encombrement (3 mm x 5 mm). On pourrait aussi utiliser un capteur sensible à une pression ou à une rotation (un potentiomètre rotatif par exemple) d'une coquille par rapport à l'autre.

On a représenté à la figure 5 le film 7 développé dans un plan. Les électrodes 5 et 6 sont formées sur une face du film et sont électriquement reliées au module électronique 3 fixé sur l'autre face, par des connexions 5a,6a respectivement, formées sur et à travers le film en même temps que les électrodes. Le capteur 12 est de même relié au module électronique par des connexions non représentées sur la figure 5. Sur cette figure, on a représenté un organe indicateur 13, tel qu'une diode témoin montée sur le module 3 pour signaler à l'utilisateur que le dispositif est activé et qu'un courant d'assistance passe entre les deux électrodes.

Après avoir installé le dispositif sur la racine du pénis, l'utilisateur active celui-ci en actionnant un interrupteur qui peut prendre diverses formes. Sur la figure 5, celui-ci prend la forme d'une tirette 14 qui, en position, ouvre un circuit d'alimentation électrique du module. En retirant la tirette 14 du dispositif, l'utilisateur ferme le circuit d'alimentation et le module 3 produit le courant d'assistance ionophorétique qui doit passer entre les électrodes 5 et 6. Bien entendu, quand on utilise un tel courant d'assistance, les réservoirs 1 et 2 doivent être imprégnés d'une solution d'une forme ionisée des molécules de principe actif précitées.

D'autres types d'interrupteurs pourraient être utilisés, à glissière, à poussoir, éventuellement munis de points de rupture de manière à ne pouvoir être actionnés qu'une seule fois. La réutilisation du dispositif est alors impossible, ce qui peut être souhaité pour des raisons d'hygiène et/ou pour des raisons pharmacologiques.

L'interrupteur pourrait encore comprendre une tirette, telle que la tirette 14, agencée pour s'escamoter automatiquement lors de la mise en place du dispositif sur le pénis, par exemple du fait de l'écartement des coquilles du dispositif de la figure 1, qui intervient lors de cette mise en place.

On notera que le dispositif suivant l'invention pourrait être avantageusement associé à un préservatif solidaire du dispositif ou vendu avec lui, sous le même emballage.

On se réfère maintenant aux figures 3 et 4 du dessin annexé où l'on a représenté un deuxième mode de réalisation du dispositif suivant l'invention, qui se distingue essentiellement du précédent par la forme du bracelet de support et le matériau utilisé pour constituer ce bracelet. Sur les figures 3 et 4 des références numériques identiques à des références déjà utilisées aux figures 1 et 2 désignent des organes ou éléments identiques ou similaires. Sur la vue éclatée de la figure 3, il apparaît que le bracelet de support prend une forme cylindrique et supporte intérieurement le film 7 sur lequel sont fixés des réservoirs 1, 2 et le module électronique 3, comme dans le mode de réalisation précédent. Le bracelet 8' est réalisé dans un matériau souple extensible, tricoté, tissé ou non tissé. Dans le cas d'un matériau tricoté, un tricot du type jersey, fortement extensible dans une direction, est préféré. Dans le cas d'un matériau non tissé, on utilisera de préférence des matériaux constitués avec des fibres intrinsèquement élastiques, du type "Lycra" (marque déposée) ou autre. Des matériaux préférés sont ceux vendus sous les marques Urgoband et Surgifix déposées par la demanderesse et par la société ADIFARM, respectivement.

La fixation des divers organes sur le bracelet 8', dans le montage représenté à la figure 4, peut être réalisée par collage ou avec des dispositifs d'accrochage du type "Velcro" (marque déposée), si l'on souhaite réutiliser le module électronique 3, par exemple. Avec le bracelet du mode de réalisation des figures 3 et 4, le capteur 12 utilisé sera de préférence une jauge extensométrique collée sur le bracelet dans la partie de celui-ci qui est libre de tout autre organe, comme représenté à la figure 4. La liaison électrique de la jauge avec le module électronique est alors assurée par des conducteurs 15a, 15b conformés comme représentés à la figure 6, analogue par ailleurs à la figure 5, les conducteurs 15a, 15b étant formés sur le film 7 en même temps que les électrodes 5, 6.

On se réfère maintenant à la figure 7 du dessin annexe pour décrire un premier mode de réalisation des moyens d'assistance ionophorétique qui délivrent un courant électrique aux électrodes 5, 6. A l'exception du capteur 12, tous les moyens représentés sur la figure 7 sont incorporés au module électronique 3. C'est ainsi que celui-ci comprend, outre les piles 16 et l'interrupteur de mise en route 17 déjà mentionnés, un circuit de commande 18, une alimentation à découpage 19, un dispositif électronique à seuil 20 et un générateur de courant 21. La fermeture de l'interrupteur 17 active le circuit de commande 18 et l'alimentation à découpage 19. Cette dernière permet d'alimenter le générateur de courant 21, du type "montage de Howland" par exemple, avec une tension de quelques dizaines de volts, à partir des quelques volts débités par les piles 16. L'alimentation du générateur 21 est commandée par le circuit 18 par l'intermédiaire d'un interrupteur électronique 22, le circuit 18 étant dûment programmé pour déterminer l'intensité et la forme d'onde du courant débité par le générateur 21, comme cela est connu dans la technique. C'est ainsi que le courant débité est typiquement compris entre 0 et 5 mA, en continu ou en alternatif.

Quand, au cours du traitement, le dispositif à seuil 20 reçoit du capteur 12 un signal représentatif d'un gonflement suffisant du pénis, soit un signal dépassant un seuil prédéterminé, le dispositif 18 commande l'ouverture de l'interrupteur 22. La fourniture d'un courant d'assistance au dispositif suivant l'invention est alors interrompue ce qui provoque une forte réduction ou même une annulation du flux de principe actif responsable dudit gonflement. Grâce à cette disposition, on supprime tout risque de sur-administration inutile ou dangereuse du médicament. Bien entendu des moyens de réglage du niveau du seuil peuvent être incorporés au dispositif 20.

On a représenté à la figure 8 un deuxième mode de réalisation des moyens d'assistance ionophorétique susceptibles d'être incorporés au dispositif suivant l'invention. On retrouve dans celui-ci les organes 16, 17, 18, 19 et 21 du mode de réalisation de la figure 7. Il s'en distingue en ce qu'il comprend des moyens permettant de programmer dans le temps l'administration du médicament comme il est connu dans la technique. La commande 18 comprend alors des moyens, éventuellement programmables, de calcul d'une valeur de consigne évoluant dans le temps. Cette consigne est comparée en 23 au signal délivré par le capteur et le signal d'erreur ε assure la régulation du courant fourni aux électrodes du dispositif par le générateur de courant 21.

Il apparaît maintenant que le dispositif suivant l'invention permet bien d'atteindre les buts que l'on s'était fixés, à savoir assurer un traitement commode, indolore et sans danger de l'impuissance érectile masculine.

Bien entendu, l'invention n'est pas limitée aux modes de réalisation décrits et représentés qui n'ont été donnés qu'à titre d'exemple. Ainsi certaines parties du dispositif pourraient être à usage unique (les électrodes et les réservoirs par exemple) et d'autres être réutilisables (le module électronique 3 par exemple). De même, le circuit imprimé sur lequel sont fixés les composants du module électronique peut être formé directement sur une face du film 7, celui-ci portant les électrodes 5 et 6 sur son autre face. Les supports de circuits disponibles dans le commerce sous les marques déposées Kapton ou Bendflex, par exemple, peuvent alors servir pour constituer le film 7.

## Revendications

1. Dispositif d'administration transdermique d'un médicament pour le traitement de l'impuissance érectile masculine, comprenant au moins un réservoir (1, 2) hydratable avec une solution du médicament et conçu pour s'appliquer sur la peau du pénis, un moyen en forme de bracelet (8; 8') sensiblement cylindrique à diamètre élastiquement expansible pour supporter et plaquer le réservoir hydraté (1, 2) contre ladite peau pendant toute la durée du traitement, des moyens (3, 5, 6), également supportés par le bracelet (8; 8') pour assurer une assistance par ionophorèse du passage du médicament à travers la peau du patient, caractérisé en ce qu'il comprend un capteur (12) fixé sur le bracelet (8; 8') et sensible à l'expansion de celui-ci pour fournir un signal représentatif de cette expansion aux moyens d'assistance (3, 5, 6) de manière à assurer une commande de ceux-ci en fonction de l'expansion du bracelet (8, 8').

2. Dispositif conforme à la revendication 1, caractérisé en ce qu'il comprend des moyens (20, 22) pour mettre hors service les moyens d'assistance (3, 5, 6) quand le signal reçu du capteur (12) dépasse un seuil prédéterminé.

3. Dispositif conforme à la revendication 2, caractérisé en ce que les moyens d'assistance (3, 5, 6) comprennent des moyens de régulation (12, 18, 23) d'un courant électrique d'assistance, le capteur (12) formant partie de ces moyens de régulation (12, 18, 23) pour assurer le suivi d'une valeur de consigne par le courant d'assistance.

4. Dispositif conforme à l'une quelconque des revendications 1 à 3, caractérisé en ce que le capteur (12) est constitué par une jauge de contrainte fixée sur le bracelet (8, 8') de manière à être sensible à l'expansion de celui-ci.

5. Dispositif conforme à l'une quelconque des revendications 1 à 4, caractérisé en ce que le bracelet (8) comprend deux parties rigides (9a, 9b) incurvées articulées l'une sur l'autre autour d'un axe (10) définissant une génératrice de la surface cylindrique du bracelet, des moyens élastiques (11a, 11b) montés sur cet axe (10) chargeant les deux parties l'une vers l'autre dans le sens de la fermeture du bracelet (8).

6. Dispositif conforme à l'une quelconque des revendications 1 à 4, caractérisé en ce que ledit bracelet (8) est réalisé en un matériau élastiquement déformable.

7. Dispositif conforme à la revendication 1, caractérisé en ce qu'il comprend une source (16) d'énergie électrique d'alimentation des moyens d'assistance par ionophorèse du passage du médicament dans le corps et un interrupteur électrique (14; 17) actionné par l'utilisateur du dispositif pour activer lesdits moyens d'assistance.

8. Dispositif conforme à la revendication 7, caractérisé en ce que l'interrupteur (14) est conçu pour n'être actionnable qu'une fois.

9. Dispositif conforme à la revendication 7 ou 8, caractérisé en ce que les moyens d'assistance (3,5,6) comprennent au moins des première (5) et deuxième (6) électrodes en contact direct ou électrique avec un réservoir (1) et la peau du patient, respectivement, et un module électronique (3) alimenté par la source d'énergie électrique pour faire passer un courant entre les deux électrodes.

10. Dispositif conforme à la revendication 9, caractérisé en ce que le module électronique (3) comprend une alimentation à découpage (19) pour élever la tension délivrée par la source d'énergie électrique, et un générateur de courant (21) alimenté par la tension délivrée par l'alimentation à découpage (19) pour faire passer un courant entre les deux électrodes (5, 6).

11. Dispositif conforme à l'une quelconque des revendications 9 et 10, caractérisé en ce qu'il comprend un film souple (7) portant les deux électrodes sur une face et le module électronique (3) sur l'autre face, les composants de ce dernier étant implantés sur un circuit imprimé directement sur cette face.

12. Dispositif conforme à l'une quelconque des revendications 7 à 11, caractérisé en ce qu'il comprend un indicateur (13) qui émet un signal lumineux quand les moyens d'assistance (3, 5, 6) sont activés.

13. Dispositif conforme à l'une quelconque des revendications précédentes, caractérisé en ce que le principe actif du médicament administré est pris dans le groupe formé par : la moxisylite et ses sels, l'agoniste du polypeptide intestinal vasoactif, la papavérine seule ou associée à la phentolamine, l'agoniste de la prostaglandine E1, la prostaglandine E1, l'agoniste de l'adrénorécepteur Béta 2, l'antagoniste de la hydroxytryptamine 2, l'antagoniste de l'adrénorécepteur Alpha 2, l'agoniste de la dopa d2, l'inhibiteur de la capture de l'hydroxytryptamine, la phénoxybenzamine, l'acétylcholine, la sydnonimine et dérivés, la testostérone, les vasodilatateurs périphériques, le minoxidil et ses dérivés, l'alprostadil et des récepteurs histaminiques H2 et H3 agoniste comme l'impromidine.

14. Dispositif conforme à l'une quelconque des revendications précédentes, associé à un préservatif.

## Claims

1. Device for percutaneous administration of a medicament for treating male impotence, comprising at least one reservoir (1, 2) which can he moisturized with a solution of the medicament and is designed to bear on the skin of the penis, means in the form of a substantially cylindrical bracelet (8, 8'), of elastically expansible diameter, for supporting and pressing the moisturized reservoir (1, 2) against said skin throughout the duration of the treatment, means (3, 5, 6), likewise supported by the bracelet (8, 8'), for assisting the passage of the medicament through the patient's skin by ionophoresis, characterized in that it comprises a sensor (12) which is fixed on the bracelet (8, 8') and is sensitive to the expansion of the latter in order to deliver a signal, representative of this expansion, to the means of assistance (3, 5, 6), in such a way as to ensure that these means are controlled as a function of the expansion of the bracelet (8, 8').

2. Device as claimed in claim 1, characterized in that it comprises means (20, 22) for cutting off the means of assistance (3, 5, 6) when the signal received from the sensor (12) exceeds a predetermined threshold.

3. Device as claimed in claim 2, characterized in that the means of assistance (3, 5, 6) comprise means (12, 18, 23) for regulating an electrical assist current, the sensor (12) forming part of these regulating means (12, 18, 23) to ensure the monitoring of a reference variable by the assist current.

4. Device as claimed in any one of claims 1 to 3, characterized in that the sensor (12) consists of a strain gauge which is fixed on the bracelet (8, 8') so as to he sensitive to the expansion thereof.

5. Device as claimed in any one of claims 1 to 4, characterized in that the bracelet (8) comprises two inwardly curving, rigid parts (9a, 9b) which are articulated on one another about an axis (10) defining a generatrix of the cylindrical surface of the bracelet, elastic means (11a, 11b) mounted on this axis (10) stressing the two parts toward one another in the direction of closure of the bracelet (8).

6. Device as claimed in any one of claims 1 to 4, characterized in that said bracelet (8) is made of an elastically deformable material.

7. Device as claimed in claim 1, characterized in that it comprises a source (16) of electrical energy supplying the means for assisting the passage of the medicament into the body by ionophoresis, and an electrical switch (14; 17) operated by the user of the device in order to activate said means of assistance.

8. Device as claimed in claim 7, characterized in that the switch (14) is designed to he operated only once.

9. Device as claimed in claim 7 or 8, characterized in that the means of assistance (3, 5, 6) comprise at least first (5) and second (6) electrodes in direct or electrical contact with a reservoir (1) and the patient's skin, respectively, and an electronic module (3) supplied by the source of electrical energy so as to pass a current between the two electrodes.

10. Device as claimed in claim 9, characterized in that the electronic module (3) comprises a switching power supply (19) for increasing the voltage delivered by the source of electrical energy, and a current generator (21) supplied with the voltage supplied by the switching power supply (19) in order to pass a current between the two electrodes (5, 6).

11. Device as claimed in either of claims 9 and 10, characterized in that it comprises a flexible film (7) bearing the two electrodes on one face and the electronic module (3) on the other face, the constituent parts of said module being implanted on a circuit printed directly on this face.

12. The device as claimed in any one of claims 7 to 11, comprising an indicator (13) which emits a light signal when the means of assistance (3, 5, 6) are activated.

13. Device as claimed in any one of the preceding claims, wherein the active principle of the administered medicament is taken from the group formed by: moxisylyte and its salts, the agonist of vasoactive intestinal polypeptide, papaverine, alone or in combination with phentolamine, the agonist of prostaglandin E1, prostaglandin E1, the agonist of the beta-2 adrenergic receptor, the antagonist of 2-hydroxytryptamine, the antagonist of the alpha-2 adrenergic receptor, the agonist of dopa d2, the hydroxy-tryptamine re-uptake inhibitor, phenoxybenzamine, acetylcholine, sydnonimine and derivatives, testosterone, peripheral vasodilators, minoxidil and its derivatives, alprostadil, and histamine H2 and H3 receptor agonists, such as impromidine.

14. Device as claimed in any one of the preceding claims, combined with a condom.

## Patentansprüche

1. Vorrichtung zur transdermalen Verabreichung eines Medikaments zur Behandlung der männlichen erektilen Impotenz, bestehend aus mindestens einem mit einer Lösung des Medikaments hydratisierbaren Behälter (1, 2), der so ausgelegt ist, daß er an die Haut des Penis angelegt werden kann, einer Einrichtung in Form eines im wesentlichen zylindrischen Reifens (8; 8') mit einem elastisch ausdehnbaren Durchmesser zum Tragen und Anlegen des hydratisierten Behälters (1, 2) an die Haut während der ganzen Dauer der Behandlung und aus ebenfalls von dem Reifen (8; 8') getragenen Einrichtungen (3, 5, 6) zum Unterstützen des Durchgangs des Medikaments durch die Haut des Patienten durch Iontophorese, dadurch gekennzeichnet, daß sie einen Fühler (12) aufweist, der auf dem Reifen (8; 8') befestigt ist und für dessen Ausdehnung empfindlich ist, um ein für diese Ausdehnung repräsentatives Signal den Unterstützungseinrichtungen (3, 5, 6) zu liefern, so daß, diese in Abhängigkeit von der Ausdehnung des Reifens (8, 8') gesteuert werden.

2. Vorrichtung nach Anspruch 1, dadurch gekennzeichnet, daß sie Einrichtungen (20, 22) aufweist, um die Unterstützungseinrichtungen (3, 5, 6) abzuschalten, wenn das vom Fühler (12) empfangene Signal eine zuvor festgelegte Schwelle überschreitet.

3. Vorrichtung nach Anspruch 2, dadurch gekennzeichnet, daß die Unterstützungseinrichtungen (3, 5, 6) Einrichtungen (12, 18, 23) zur Regelung eines elektrischen Unterstützungsstroms aufweisen, von denen der Fühler (12) einen Teil bildet, um zu gewährleisten, daß der Unterstützungsstrom einen Sollwert einhält.

4. Vorrichtung nach einem der Ansprüche 1 bis 3, dadurch gekennzeichnet, daß der Fühler (12) aus einem Dehnungsmeßstreifen besteht, der an dem Reifen (8, 8') so befestigt ist, daß er für dessen Ausdehnung empfindlich ist.

5. Vorrichtung nach einem der Ansprüche 1 bis 4, dadurch gekennzeichnet, daß der Reifen (8) aus zwei gekrümmten starren Teilen (9a, 9b) besteht, die über eine Achse (10) aneinander angelenkt sind, die eine Erzeugende der zylindrischen Fläche des Reifens bildet, wobei auf dieser Achse (10) montierte elastische Einrichtungen (11a, 11b) die beiden Teile in Richtung der Schließung des Reifens (8) aufeinander zu beaufschlagen.

6. Vorrichtung nach einem der Ansprüche 1 bis 4, dadurch gekennzeichnet, daß der Reifen (8) aus einem elastisch verformbaren Werkstoff besteht.

7. Vorrichtung nach Anspruch 1, dadurch gekennzeichnet, daß sie eine elektrische Energiequelle (16) zur Versorgung der Einrichtungen zur Unterstützung des Durchgangs des Medikaments in den Körper durch Iontophorese und einen durch den Benutzer betätigten elektrischen Schalter (14; 17) zur Aktivierung der Untestützungseinrichtungen aufweist.

8. Vorrichtung nach Anspruch 7, dadurch gekennzeichnet, daß der Schalter (14) so ausgelegt ist, daß er nur einmal betätigt wird.

9. Vorrichtung nach Anspruch 7 oder 8, dadurch gekennzeichnet, daß die Unterstützungseinrichtungen (3, 5, 6) mindestens eine erste Elektrode (5) und eine zweite Elektrode (6) aufweisen, die mit einem Behälter (1) bzw. mit der Haut des Patienten in direktem oder elektrischem Kontakt sind, sowie ein durch die elektrische Energiequelle gespeistes elektronisches Modul (3), um zwischen den beiden Elektroden einen Strom fließen zu lassen.

10. Vorrichtung nach Anspruch 9, dadurch gekennzeichnet, daß das elektronische Modul (3) eine Zerhackungsversorgung (19) zur Erhöhung der von der elektrischen Energiequelle gelieferten Spannung und einen Stromgenerator (21) aufweist, der mit der von der Zerschneidungsversorgung (19) gelieferten Spannung versorgt wird, um zwischen den beiden Elektroden (5, 6) einen Strom fließen zu lassen.

11. Vorrichtung nach einem der Ansprüche 9 und 10, dadurch gekennzeichnet, daß sie eine flexible Folie (7) aufweist, die die beiden Elektroden auf einer Seite und das elektronische Modul (3) auf der anderen Seite trägt, wobei dessen Bauelemente in eine gedruckte Schaltung direkt auf dieser Seite eingegliedert sind.

12. Vorrichtung nach einem der Ansprüche 7 bis 11, dadurch gekennzeichnet, daß sie einen Anzeiger (13) aufweist, der ein Lichtsignal sendet, wenn die Unterstützungsmittel (3, 5, 6) aktiviert sind.

13. Vorrichtung nach einem der vorhergehenden Ansprüche, dadurch gekennzeichnet, daß der Wirkstoff des verabreichten Medikaments aus der Gruppe ausgewählt ist, die aus folgenden Substanzen besteht: Moxisylit und seine Salze, der Agonist des vasoaktiven intestinalen Polypeptids, Papaverin allein oder verbunden mit Phentolamin, der Agonist des Prostaglandins E1, Prostaglandin E1, der Agonist des adrenergen Beta-2-Rezeptors, der Antagonist von Hydroxytryptamin 2, der Antagonist des adrenergen Alpha-2-Rezeptors, der Agonist von Dopa d2, der Inhibitor der Bindung von Hydroxytryptamin, Phenoxybenzamin, Acetylcholin, Sydnonimin und Derivate, Testosteron, die peripheren Vasodilatatoren, Minoxidil und seine Derivate, Alprostadil und ein Agonist der Histaminrezeptoren H2 und H3 wie Impromidin.

14. Vorrichtung nach einem der vorhergehenden Ansprüche, die einem Präservativ zugeordnet ist.
